# EUROPEAN PATENT APPLICATION

(11) **EP 4 230 211 A1**
(43) Date of publication of application: **23.08.2023**
(21) Application number: 20964393.1
(22) Date of filing: 04.12.2020
(51) Int. Cl.: A61K 31/573, A61K 31/4545, A61P 37/08

(54) **PHARMACEUTICAL COMBINATION OF A CORTICOSTEROID AND AN ANTIHISTAMINE FOR THE TREATMENT AND CONTROL OF THE INFLAMMATORY COMPONENT OF ALLERGIC PROCESSES**

(71) Applicant: Laboratorios Silanes, S.A. de C.V., Mexico, 11000 (MX)
(72) Inventor: MUÑOZ MARTINEZ, Cecilia Jannette, Ciudad de México, 11000 (MX); FARFÁN SALAZAR, Claudia Delfina, Ciudad de México, 11000 (MX); ESPINOZA LEÓN, Sixto Serafín, Ciudad de México, 11000 (MX); GONZÁLEZ CANUDAS, Jorge Alejandro, Ciudad de México, 11000 (MX); OLLERVIDES RUBIO, Paola Yazmín, Ciudad de México, 11000 (MX)
(74) Representative: Araujo, Daniel
(86) International application number: PCT/MX2020/050048
(87) International publication number: WO 2022/119428

(57) **Abstract**

The present invention combines in only one dosage unit the drugs desloratadine and betamethasone, which represents a set of important technological challenges due to the physicochemical properties and the difference in dosage to ensure the obtaining of a stable product for the treatment of allergic and inflammatory processes in a subject.

## Description

### TECHNICAL FIELD

This invention pertains to the field of the pharmaceutical chemistry and relates to combination pharmaceutical compositions for the treatment of allergic and inflammatory processes in a subject.

### BACKGROUND

In the state of the art, we find patent US4659716, which holder is the company SCHERING CORP and describes the drug desloratadine, a pharmaceutical composition containing it, as well as a method for treating allergic reactions.

Successively, patent MX 219756, of the same company describes crystalline forms I and II of decarbonylethoxyloratadine, characterized by an X-ray powder diffraction pattern, as well as pharmaceutical compositions respectively containing crystalline forms I and II.

The use of decarboethoxyloratadine to treat allergic rhinitis, urticaria, and allergic asthma in humans has been described in Mexican patents MX 199854, MX 267849, and MX 277173, from the company SEPRACOR, INC. Subsequently, patent MX244854, having the same title, discloses in its main claim a lactose-free pharmaceutical composition for the treatment of histamine-induced disorders, characterized in that it is in the form of a tablet comprising a therapeutically effective amount of decarboethoxyloratadine, or a pharmaceutically acceptable salt thereof, which has been granulated with a pharmaceutically acceptable inert carrier.

Similarly, MERCK SHARP & DOHME CORP's MX 263814 patent protects the use of desloratadine and a suitable pharmaceutically acceptable inert carrier or diluent for preparing a pharmaceutical composition useful for the treatment or prevention of allergic and inflammatory conditions of the skin or airway passages in a human aged 12 years and older, wherein the pharmaceutical composition is adapted to be administrable to achieve a pharmacokinetic (pK) profile for desloratadine comprising a maximum steady-state arithmetic or geometric mean plasma concentration (C-max) of desloratadine of about 4 ng/mL, and an arithmetic or geometric mean time to reach the maximum plasma concentration (Tmax) of desloratadine of about 3 hours post-dose.

Even, the patent MX 294107 also from the company SEPRACOR, INC., protected a pharmaceutical composition comprising 0.1 to 10 mg of descarboethoxyloratadine or a pharmaceutically acceptable salt thereof and a pharmaceutically acceptable carrier.

Subsequently, international patent application WO2005/065047 claimed a stable oral composition comprising a therapeutically effective amount of desloratadine, a stabilizer selected from the group comprising an antioxidant, a pharmaceutically acceptable organic compound providing an alkaline pH, an alkali metal salt, or mixtures thereof and pharmaceutically acceptable excipients. It also mentions that the acidic excipients color and decompose Desloratadine. At 40 °C and 75%RH desloratadine exhibits color changes alone or in combination with various excipients. The color change in the active ingredient is attributed to the N-formyl impurity which is formed in the presence of a wide variety of excipients used in oral formulations. Suitable excipients include acidic excipients such as stearic acid, povidone, and crospovidone or excipients having a pH less than 7 in water, a pH in the range of 3 to 5 is preferred, such as lactose, lactose monohydrate, sodium benzoate, as well as carbonates, phosphates, silicates, and sulfates of the aluminum, magnesium, and calcium salts or mixtures thereof.

The patent "Compositions and methods for treating atopic dermatitis, angioedema, and other disorders using antihistamines and glucocorticoids" WO 00/51605 relates to compositions and methods for the treatment of atopic dermatitis, angioedema, urticaria, allergic rhinitis, and other disorders. The patent comprises a dosage form comprising an antihistamine (loratadine) and a glucocorticoid (betamethasone).

In the scientific literature the article "Association between desloratadine and prednisolone in the treatment of children with acute symptoms of allergic rhinitis: a double-blind, randomized and controlled clinical trial" mentions that the combination of antihistamines and corticosteroids are frequently used to treat allergic rhinitis. The objective of the study was to evaluate the efficacy and safety of desloratadine and prednisolone in the control of acute and severe nasal symptoms in children with moderate to severe persistence of allergic rhinitis and to compare with dexchlorpheniramine-betamethasone, concluding that the combination of desloratadine plus prednisolone was able to effectively control the symptoms of rhinitis in children, improving symptoms and nasal function.

Compared to the combination of dexchlorpheniramine plus betamethasone, it showed a similar clinical action, but with a lower incidence of adverse events and greater convenience of dosing.

As for US patent US3053865 under the name of MERCK & CO INC, it describes the drug betamethasone, as well as a method for preparing it.

Likewise, there is a description of the combination of these drugs, desloratadine and betamethasone, for example, patent application MX/a/2007/011772 of ELAN PHARMA INTERNATIONAL LTD, discloses an aerosol composition comprising at least one nanoparticulate corticosteroid and at least one antihistamine. The nanoparticulate corticosteroid has an effective average particle size of less than about 2000 nm. The antihistamine includes, among others, desloratadine and the corticosteroid includes, betamethasone, among others. The claimed composition comprises an amount of antihistamine of about 0.1 to about 10 wt% and an amount of corticosteroid of about 0.01 to about 10 wt%. The composition is formulated for oral, pulmonary, otic, rectal, ocular, ophthalmic, colonic, parenteral, intracisternal, intravaginal, intravenous, intraperitoneal, local, buccal, nasal, and topical administration. However, the composition of the present invention is not formulated with nanoparticles and the manufacturing process is different.

MERCK SHARP & DOHME CORP's Document PA/a/1999/002272 describes a pharmaceutical composition for treatment of atopic dermatitis, angioedema, urticaria, seasonal and allergic rhinitis, food and drug allergies, allergic contact dermatitis, seborrheic dermatitis, neurodermatitis, allergic asthma, ocular allergic manifestations such as conjunctivitis and iridocyclitis, allergic reactions to insect bites and stings, this composition comprising: (i) a therapeutically effective amount of one or more substantially non-sedative antihistamines or a pharmaceutically acceptable salt or solvate thereof; and (ii) a therapeutically effective amount of one or more glucocorticoids. In one form, said antihistamine is desloratadine in the range of 2 to 20 mg per dose and the glucocorticoid is betamethasone present at about 0.02-0.8 mg per dose; also in one form, the dosage form of the composition includes tablets prepared by compression or by granulation.

However, application PA/a/1999/002272 does not refer to dissolution or stability results, nor does it indicate an immediate release of the active ingredients, so the invention described herein cannot be inferred from the application of MERCK SHARP & DOHME CORP.

In addition, the formulation of this invention contains excipients such as "dibasic phosphate of anhydrous calcium", which is used to stabilize desloratadine by providing a slightly alkaline pH, and "aluminum magnesium metasilicate" which facilitates the incorporation of betamethasone. These are components that confer greater stability to the formulation of the present invention.

There is a scientific article entitled "Bioequivalence of a Fixed Dose Combination of Desloratadine/Betamethasone Tablets (Oradus Beta) in Healthy Human Volunteers."

The sponsor of this trial is the Pharmaceutical Research Unit, Clinical Evaluation Centre.

The study was conducted to compare the pharmacokinetics of the desloratadine and betamethasone tablet combination in 40 healthy human volunteers after a single oral dose in a two-period, two-treatment, two-sequence crossover study group. The study protocol was prepared in accordance with the requirements set out in the EMA guidance for conducting bioequivalence studies. Reference (Frenaler Cort 5 mg desloratadine / 0.6 mg betametasone-coated tablets, Roemmers S.A.I.C.F., Argentina) and test (Oradus β 5 mg desloratadine / 0.6 mg betamethasone-coated tablets, Pharmaline, Lebanon). The resulting data demonstrated that when administered as a combination of fixed dose, the pharmacokinetics of desloratadine and betamethasone are bioequivalent and well tolerated. The link of the study in question is:https://www.longdom.org/open-access/bioequivalence-of-a-fixed-dose-combination-of-desloratadinebetamethasonetablets-oradus-beta-in-healthy-human-volunteers-ibb-1000301.pdf

However, the concentration of the active substance betamethasone is different from that of the present invention.

In addition, there are products currently on the market that involve the combination of desloratadine/betamethasone, for example: HEXALER CORT ^{®} of INVESTI-FARMA S.A.; FRENALER CORT ^{®} of Laboratories Roemmers SAICF; DELORAT B ^{®} of Dr. LAZAR and CIA S.A. QUIMICA E INDUSTRIAL, which dosage form is in coated tablets [5.0 mg desloratadine / 0.6 mg betamethasone];

However, the present invention solves a set of important technological challenges resulting from the physicochemical properties and the difference in dosage to ensure the obtaining of a stable product in a single dosage unit of immediate release of the drugs desloratadine and betamethasone, demonstrating a synergistic effect for the control and treatment of allergic and inflammatory processes.

### SUMMARY OF THE INVENTION

The present invention relates to solid, stable, and immediate-release pharmaceutical compositions comprising desloratadine and betamethasone, or a pharmaceutically acceptable salt thereof, for the treatment of allergic and inflammatory processes in a subject.

A form of the invention consists of a pharmaceutical composition comprising: (a) desloratadine, or an equivalent amount of a salt thereof, in a pharmaceutically acceptable amount of 5.00 mg and (b) betamethasone, or an equivalent amount of a salt thereof, in a pharmaceutically acceptable amount of 0.25 mg, and (c) a pharmaceutically acceptable amount of excipients and / or acceptable pharmaceutical vehicles.

Acceptable pharmaceutical excipients and/or vehicles include diluents, disintegrants, adsorbents, surfactants, lubricants, solvents, and coatings. In an additional form of the invention, the coating is a barrier against moisture.

In another form of the invention the composition is in the form of pastilles, tablets, granules, lozenges, pills. Preferably, it is in the form of a tablet and/or coated pill.

Another aspect of the invention comprises the use of the pharmaceutical composition for the manufacture of a drug product useful in the treatment of allergic and inflammatory processes in a subject, wherein said allergic and inflammatory processes are selected from the group of: severe symptoms of atopic dermatitis, angioedema, urticaria, seasonal and perennial allergic rhinitis, allergic food, and drug reactions, allergic asthma, and allergic reactions secondary to insect bites.

### BRIEF DESCRIPTION OF FIGURES

**Figure 1****.** Betamethasone dissolution profile.
**Figure 2****.** Desloratadine dissolution profile reference vs test.
**Figure 3****.** Desloratadine Pharmacokinetic profile, normal scale.
**Figure 4****.** Desloratadine Pharmacokinetic profile, semilogarithmic scale.
**Figure 5****.** Betamethasone pharmacokinetic profile, normal scale.
**Figure 6****.** Betamethasone pharmacokinetic profile, semilogarithmic scale.

### DETAILED DESCRIPTION OF THE INVENTION

### Definitions

Pharmaceutically acceptable salt: the term "pharmaceutically acceptable salt" of a given compound refers to salts that retain the biological efficacy and properties of the given compound, and that are not biologically, or otherwise, undesirable (P. Heinrich Stahl and Camille G. Wermuth (Eds.) Pharmaceutical Salts Properties, Selection, and Use (International Union of Pure and Applied Chemistry), Wiley-VCH; 2nd Revised Edition (May 16, 2011)).

Excipient: it is the ingredient that is part of the present pharmaceutical composition, among them are diluents, disintegrants, surfactants, lubricants, coating, adsorbents, among others.

Stability. It is the ability of a pharmaceutical product to retain its chemical, physical, microbiological, and biopharmaceutical properties within specified limits throughout its shelf life.

The present invention relates to solid, stable, immediate-release pharmaceutical compositions with synergistic effect, maintaining the dissolution and bioavailability, of an antihistamine and a glucocorticoid, which are administered in a single oral pharmaceutical form as a therapeutic agent for the treatment of severe symptoms of atopic dermatitis, angioedema, urticaria, seasonal and perennial allergic rhinitis, allergic food and drug reactions, allergic asthma, and allergic reactions secondary to insect bites.

The present invention also relates to the manufacture of such compositions of an antihistamine and a glucocorticoid for oral administration. Particularly, the antihistamine is selected from desloratadine, and the glucocorticoid is selected from betamethasone. The process allows to obtain a pharmaceutical composition of easy administration in a single dose.

The composition of the present invention comprises: (a) desloratadine, or an equivalent amount of a salt thereof, in a pharmaceutically acceptable amount of 5.00 mg and (b) betamethasone, or an equivalent amount of a salt thereof, in a pharmaceutically acceptable amount of 0.25 mg, and (c) a pharmaceutically acceptable amount of excipients and / or acceptable pharmaceutical vehicles.

The pharmaceutical composition can be employed for the manufacture of a drug useful in the treatment of allergic and inflammatory processes in a subject.

In one form, the composition of the present invention comprises desloratadine, preferably in its free form.

In a preferred form, the pharmaceutically acceptable amount of the drug desloratadine is adjusted according to the titration.

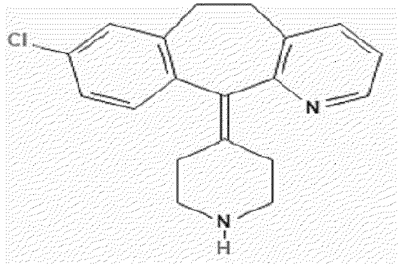

In another form, the composition of the present invention comprises betamethasone, preferably in its free form.

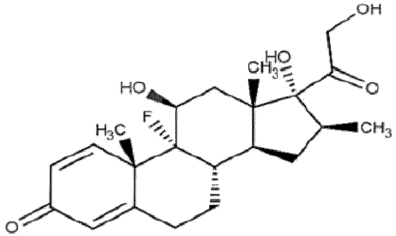

The pharmaceutical composition of the present invention comprises excipients and / or acceptable pharmaceutical vehicles and comprising but not limited to: diluents, disintegrants, adsorbents, surfactants, lubricants, solvents, and coatings.

Examples of pharmaceutically acceptable diluents, whose known function in the prior art is to adjust and maintain the constant weight of the tablet, include, but are not limited to, cellulose derivatives, such as microcrystalline cellulose PH102, phosphate derivatives such as dibasic calcium phosphate, starch derivatives such as pregelatinized starch and cornstarch, as well as, mannitol, xylitol, maltitol, lactitol, sorbitol, sucrose, or a combination thereof.

In a preferred form, the pharmaceutical composition of the invention contains as a diluent microcrystalline cellulose PH102 in a pharmaceutically acceptable amount in the range of 20 to 90%; pregelatinized starch in a pharmaceutically acceptable amount in the range of 5.0 to 75% and dibasic calcium phosphate anhydrous in a pharmaceutically acceptable amount in the range of 10 to 30%.

Pharmaceutically acceptable disintegrants include, but are not limited to, cellulose derivatives such as hydroxypropyl cellulose, carboxymethylcellulose; polyvidone derivatives such as crospovidone and copovidone; starch derivatives such as pregelatinized starch, sodium starch glycolate, corn starch, and modified potato starch. In one form, the disintegrator is preferably crospovidone, which is selected in the present invention in a pharmaceutically acceptable amount in the range of 2.0 to 5.0%.

Examples of pharmaceutically acceptable adsorbents include, but are not limited to aluminum magnesium metasilicate, aluminum derivatives such as aluminum hydroxide, aluminum oxide, aluminum phosphate; clays or earths such as atalpugite, betonite, hectorite, Kaolin, pectin; silica derivatives such as calcium silicate, colloidal silicon dioxide, aluminum silicate and magnesium; and cellulose derivatives such as microcrystalline cellulose.

In one form, the adsorbent is preferably aluminum magnesium metasilicate, which is selected in the present invention for its flow properties and ability to adsorb particles, in a pharmaceutically acceptable amount in the range of 0.5 to 90%.

Examples of pharmaceutically acceptable surfactants may be such as poloxamers, polyethylene glycol, cyclodextrins, and meglumine. In one form, the surfactant is preferably sodium lauryl sulfate in a pharmaceutically acceptable amount in the range of 0.5 to 2.5%.

Examples of pharmaceutically acceptable lubricants include, but are not limited to, magnesium stearate, zinc stearate, calcium stearate, stearic acid, monostearate, sodium stearyl fumarate; talc and sulfated derivatives such as magnesium laureth sulfate. In one form, the lubricant is preferably sodium stearyl fumarate in a pharmaceutically acceptable amount in the range of 0.5 to 2.0%.

On the other hand, the composition of the present invention may contain coatings. In one form, the moisture barrier coating is preferably opadry, such as, for example, OPADRY YS-1-7003 WHITE. in a pharmaceutically acceptable amount of 3.85%.

The solid pharmaceutical composition is preferably in the form of tablet and / or tablet coated for its accuracy of dose in addition to being an accepted dosage form for its easy administration.

The combination of desloratadine, betamethasone, and pharmaceutically acceptable excipients represents a set of important technological challenges due to the physicochemical properties of the drugs, the appropriate selection of excipients and the manufacturing conditions, as well as the difference in dose to ensure the obtaining of a stable product in the development of pharmaceutical compositions. A very important role in relation to the release of the drug, the rate of absorption, and amount absorbed in the body.

Thus, the present invention describes an optimized manufacturing process that allows the combined manufacture of the drugs desloratadine and betamethasone in a single solid, stable, immediate-release dosage form.

The production process of the tablets was based on the selection of unit operations, as well as the order and execution time to control the different physicochemical properties of the drugs. The technological challenge lies in stabilizing desloratadine since it degrades in humidity and temperature conditions. The absorption of light and moisture is moderated through the use of excipients that allow the protection of drugs without affecting their release and secondary materials that ensure their stability.

It is determined to carry out a process of direct compression and subsequent coating avoiding conditions that could affect the stability of the composition.

In another form of the invention, the drug dissolution rate was determined.

The combination of drugs suggests an effect of relieving severe symptoms of atopic dermatitis, angioedema, urticaria, allergic rhinitis, with respect to the use in isolation and may also shorten the duration of treatment and reduce adverse effects.

The expert in the art will find that multiple variations and modalities are possible in the realization of the present invention without departing from the spirit and scope of it to ensure the proper functioning of the product and meet the required quality characteristics.

Below, the manufacturing process of the coated tablets comprising desloratadine and betamethasone of the present invention are presented including but not limited to:

### Examples

### Example 1. Manufacturing process for tablet production

1. Mix Drug 1 (corticosteroid) and 10% adsorbent for 3 minutes.
2. Add 30% adsorbent to mixture 1 and mix for 3 minutes.
3. Add 60% adsorbent to mixture 2 and mix for 3 minutes.
4. Add drug 2 (antihistamine) to the mixture of step 3 and mix for 5 minutes.
5. To the mixing of step 4 add disintegrant and 32% pregelatinized starch and mix for 5 minutes.
6. To the mixing of point 5 add surfactant and 68% pregelatinized starch and mix for 5 minutes.
7. Sift the mix of point 6, microcrystalline cellulouse PH 102 and calcium dibasic phosphate
8. Mix the sift obtained from step 7 for 5 minutes.
9. Add the lubricating agent to the mixture of step 8, which was previously sieved and mix for 3 minutes.
10. Compress.
11. Perform the coating of the tablets obtained in step 10.

### Example 2. Pharmaceutical composition of desloratadine in combination with betamethasone

**Table 1. Pharmaceutical composition of desloratadine in combination with betamethasone**

| mg/Tablet | Component | Function |
|---|---|---|
| 0.25 | Betamethasone | Drug |
| 5.00 | Desloratadin | Drug |
| 78.75 | Microcrystalline cellulose PH102 | Diluent |
| 40.00 | Pregelatinized starch | Diluent |
| 5.00 | Magnesium oxide | Diluent |
| 7.00 | Sodium croscarmellose | Desintegrant |
| 5.00 | Aluminum and Magnesium silicate | Adsorbent |
| 3.00 | Sodium lauryl sulphate | Surfactant |
| 1.0 | Magnesium estearate | Lubricant |
| 3.00 | Opadry YS-1-7003 White | Coating |

### Example 3. Pharmaceutical composition of desloratadine in combination with betamethasone

**Table 2. Pharmaceutical composition of desloratadine in combination with betamethasone**

| mg/Tablet | Component | Function |
|---|---|---|
| 0.25 | Betamethasone | Drug |
| 5.00 | Desloratadin | Drug |
| 47.75 | Microcrystalline cellulose PH102 | Diluent |
| 20.00 | Calcium dibasic phosphate anhydrous | Diluent |
| 5.00 | Crospovidone | Desintegrant |
| 5.00 | Aluminum and Magnesium silicate | Adsorbent |
| 1.00 | Sodium lauryl sulphate | Surfactant |
| 3.00 | Talc | Lubricant |
| 3.00 | Opadry YS-1-7003 White | Coating |

### Example 4. Preferred pharmaceutical composition of desloratadine in combination with betamethasone

**Table 3. Preferred pharmaceutical composition of desloratadine in combination with betamethasone**

| mg / Tablet | Compound | Function |
|---|---|---|
| 0.25 | Betamethasone | Drug |
| 5.00 | Desloratadine | Drug |
| 79.00 | Microcrystalline cellulose PH102 | Thinner |
| 40.00 | Pregelatinized starch | Thinner |
| 10.00 | Dibasic calcium phosphate anhydrous | Thinner |
| 7.00 | Crospovidone | Disintegrant |
| 5.00 | Aluminum magnesium metasilicate | Absorbent |
| 2.25 | Sodium lauryl sulfate | Surfactant |
| 1.50 | Sodium stearyl fumarate | Lubricant |
| 3.00 | White opadry YS-1-7003 | Covering |

### Example 5. Dissolution profile tests.

The dissolution test (test *in vitro*) serves to determine the rate (amount/time) and extent (total amount) at which a drug is released from the dosage form; in the case of the dissolution profile corresponds to the quantification at different times of the drug dissolved under standardized conditions. The importance of the dissolution test lies in the following:
a) It is a guide for the development of new formulations during the development of the product: it allows to evaluate the possible interference of the excipients or the manufacturing process on the release of the drug.
b) Process control and quality assurance: helps to ensure the continuous quality of the product and its optimization after a change in manufacturing, formulation, manufacturing site and process scaling.
c) Developmental indicator *in vivo*: it is an indicator of bioavailability, it allows to establish the correlation between the parameters *in vitro* with bioavailability results.

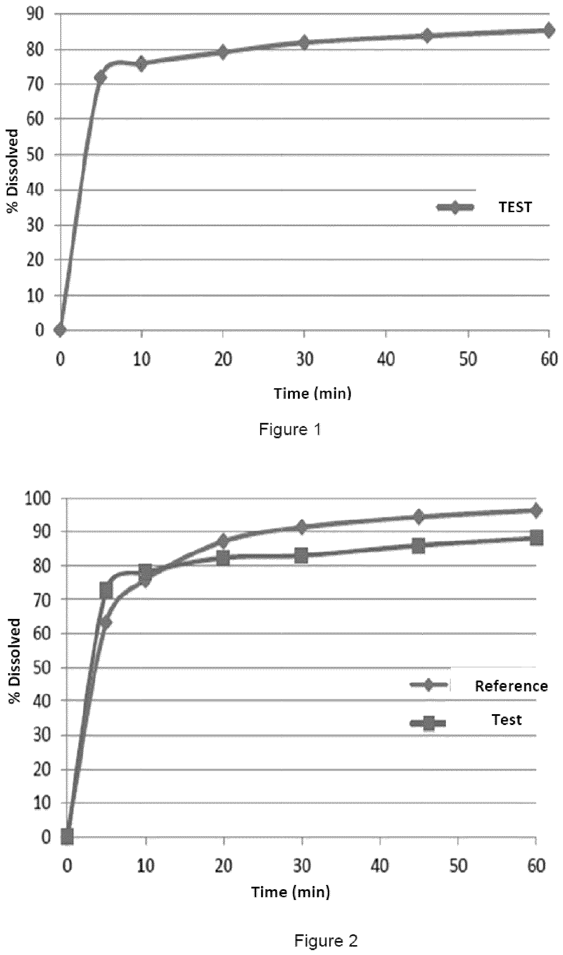

Dissolution profile tests were performed for desloratadine/betamethasone in tablets 5.00 mg /0.25 mg; the results obtained are presented below (Tables 4-7 and Figures 1 and 2):

**Table 4. Betamethasone Dissolution Results**

| Specification | | Initial | 1 month | 3 month | 6 month |
|---|---|---|---|---|---|
| Q = 70% in 45 minutes | Lot 1 | 83.5% | 83.3% | 85.6% | 85.5% |
| | Lot 2 | 80.9% | 83.9% | 84.5% | 84.8% |
| | Lot 3 | 84.3% | 84.8% | 87.5% | 84.3% |

**Table 5. Betamethasone dissolution profile.**

| t (minutes) | Test |
|---|---|
| 0 | 0.00 |
| 5 | 71.78 |
| 10 | 75.79 |
| 20 | 79.05 |
| 30 | 81.81 |
| 45 | 83.76 |
| 60 | 85.32 |

**Table 6. Desloratadine Dissolution Results**

| Specification | | Initial | 1 month | 3 month | 6 month |
|---|---|---|---|---|---|
| Q = 70% in 45 minutes | Lot 1 | 87.2% | 84.0% | 82.2% | 86.2% |
| | Lot 2 | 84.2% | 84.4% | 86.1% | 88.9% |
| | Lot 3 | 81.3% | 89.4% | 85.1% | 84.6% |

Tables 4 and 6 show the results of the dissolution test at 1, 3, and 6 months for three batches of betamethasone and desloratadine; it is observed that they comply with the specification: "Q = 70% in 45 minutes", accordingly, it is demonstrated that there is no interference between the excipients, and therefore it is ensured that the release of the active ingredients is adequate.

**Table 7. Dissolution profile of desloratadine.**

| t (minutes) | reference | test |
|---|---|---|
| 0 | 0.00 | 0.00 |
| 5 | 63.22 | 72.71 |
| 10 | 75.78 | 77.97 |
| 20 | 87.11 | 82.27 |
| 30 | 91.29 | 82.92 |
| 45 | 94.34 | 85.92 |
| 60 | 96.27 | 88.07 |

### Example 6 Stability tests

Stability testing is a means of comparing different formulations, packaging materials or manufacturing processes in short-lived experiments. As soon as the final formulation and manufacturing process are established, the manufacturer carries out a series of stability tests that will make it possible to predict the stability of the product or drug product in this case and determine its shelf life and storage conditions.

Due to the innovation carried out in the process and formulation of the drug, it was possible to obtain a product with demonstrated stability through studies in accordance with current regulations in which the quality attributes were met during the evaluation period.

Stability studies were carried out according to the current regulations of the batches of the pharmaceutical composition of desloratadine/betamethasone described in example 3. Results at 1, 3, and 6 months under the condition of (40°C ± 2°C / 75% HR ± 5% HR) are shown below:

**Table 8. Product Description Results**

| Specification | | Initial | 1 month | 3 months | 6 months |
|---|---|---|---|---|---|
| White biconvex tablet, smooth on one side and which may or may not have the "S" logo. | Lot 1 | White biconvex tablet, plain on both sides without "S" logo | White biconvex tablet, plain on both sides without "S" logo | White biconvex tablet, plain on both sides without "S" logo | White biconvex tablet, plain on both sides without "S" logo |
| | Lot 2 | White biconvex tablet, plain on both sides without "S" logo | White biconvex tablet, plain on both sides without "S" logo | White biconvex tablet, plain on both sides without "S" logo | White biconvex tablet, plain on both sides without "S" logo |
| | Lot 3 | White biconvex tablet, plain on both sides without "S" logo | White biconvex tablet, plain on both sides without "S" logo | White biconvex tablet, plain on both sides without "S" logo | White biconvex tablet, plain on both sides without "S" logo |

**Table 9. Betamethasone Content Results**

| Specification | | Initial | 1 month | 3 months | 6 months |
|---|---|---|---|---|---|
| 90.0% to 110.0% | Lot 1 | 97.9% | 97.9% | 97.9% | 97.9% |
| | Lot 2 | 98.2% | 98.2% | 98.2% | 98.2% |
| | Lot 3 | 97.2% | 97.2% | 97.2% | 97.2% |

**Table 10. Desloratadine Content Results**

| Specification | | Initial | 1 month | 3 months | 6 months |
|---|---|---|---|---|---|
| 90.0% to 110.0% | Lot 1 | 98.4% | 98.4% | 98.4% | 98.4% |
| | Lot 2 | 98.5% | 98.5% | 98.5% | 98.5% |
| | Lot 3 | 97.8% | 97.8% | 97.8% | 97.8% |

The person skilled in the art will be able to realize that various methodologies for content testing exist in the state of the art, as for example in Pharmacopoeias: FEUM, USP, British Pharmacopoeia, pharmaceutical standards and norms, so it will be evident to a person skilled in the art to follow or combine the existing cited pharmaceutical reference documents to solve and perform this methodology, wherein multiple variations are possible in the performance of this test without departing from the spirit and scope thereof to ensure the proper functioning of the product and meet the required quality characteristics.

**Table 11. Betamethasone Related Substances Results**

| Specification | | Initial | 1 month | 3 months | 6 months |
|---|---|---|---|---|---|
| Impurity E | Batch 1 | 0.00% | 0.00% | 0.00% | 0.00% |
| Not more than 1.30% | Batch 2 | 0.00% | 0.00% | 0.00% | 0.00% |
| | Batch 3 | 0.00% | 0.00% | 0.00% | 0.00% |
| Impurity F | Batch 1 | 0.00% | 0.23% | 0.24% | 0.25% |
| Not more than 0.70% | Batch 2 | 0.00% | 0.23% | 0.26% | 0.29% |
| | Batch 3 | 0.00% | 0.23% | 0.24% | 0.26% |
| Individual Impurities | Batch 1 | 0.41% | 0.45% | 0.78% | 0.86% |
| | Batch 2 | 0.53% | 0.44% | 0.91% | 0.97% |
| Not more than 1.50% | Batch 3 | 0.51% | 0.44% | 0.98% | 0.77% |
| Total Impurities | Batch 1 | 0.64% | 0.84% | 1.19% | 1.28% |
| Not more than 5.0% | Batch 2 | 0.82% | 0.83% | 1.34% | 1.44% |
| | Batch 3 | 1.11% | 0.80% | 1.40% | 1.19% |

**Table 12. Results for Desloratadine Related Substances**

| Specification | | Initial | 1 month | 3 months | 6 months |
|---|---|---|---|---|---|
| Impurity F | Batch 1 | 0.00% | 0.00% | 0.12% | 0.00% |
| Not more than 0.30% | Batch 2 | 0.00% | 0.12% | 0.12% | 0.00% |
| | Batch 3 | 0.00% | 0.12% | 0.11% | 0.00% |
| Not specified degradation products | Batch 1 | 0.00% | 0.00% | 0.00% | 0.00% |
| | Batch 2 | 0.00% | 0.00% | 0.00% | 0.00% |
| | Batch 3 | 0.00% | 0.00% | 0.00% | 0.00% |
| Not more than 0.30% | | | | | |
| Total impurities | Batch 1 | 0.00% | 0.00% | 0.12% | 0.00% |
| Not more than 1.0% | Batch 2 | 0.00% | 0.12% | 0.12% | 0.00% |
| | Batch 3 | 0.00% | 0.12% | 0.11% | 0.00% |

### Example 7. Bioavailability and pharmacokinetic parameters.

A comparative bioavailability study was conducted between 5 mg Desloratadine and 0 25 mg Betamethasone, administered alone or in combination, single dose in healthy subjects of both genders under fasting conditions.

The study design was crossover, 3 x 6 x 3 open, prospective, and longitudinal, truncated to single dose.

The test product is Desloratadine 5 mg, Betamethasone 0.25 mg, fixed combination tablet, and the reference tablet product, is Aviant^{®} made by Schering Plough, S.A. de C.V. and Betamethasone, 0.25 mg/0.5 mL Celestone^{®} 50 mg/100 mL solution made by Schering Plough, S.A. de C.V.

### Duration of treatment:

Three treatments, three periods, six sequences with an elimination period of 14 days and with a total of 24 healthy subjects of both genders, in fasting conditions.

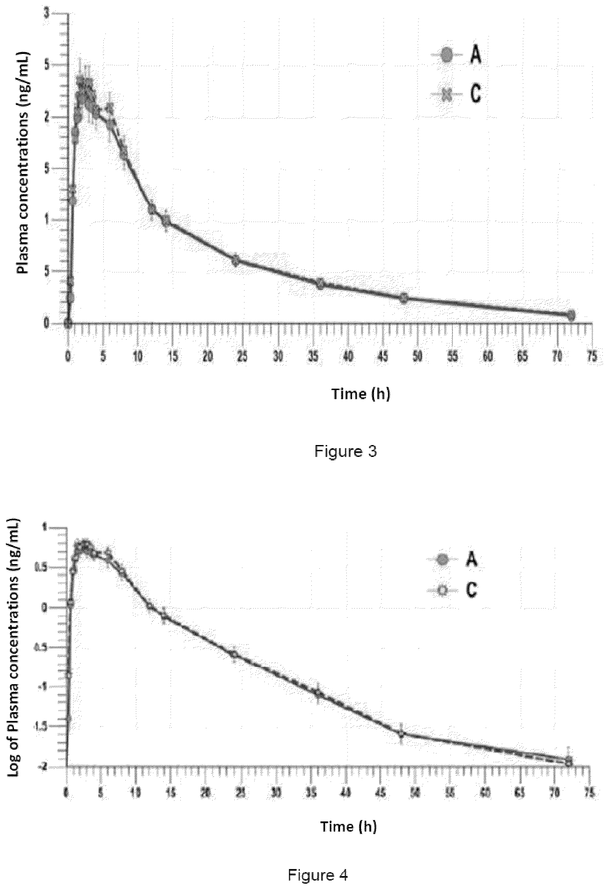

The average pharmacokinetic profile of standard error desloratadine on normal and semi-log scale for comparative A vs C. Where A is: reference drug (desloratadine); and C is: test drug (desloratadine / betamethasone) are shown in Figures 3 and 4 below.

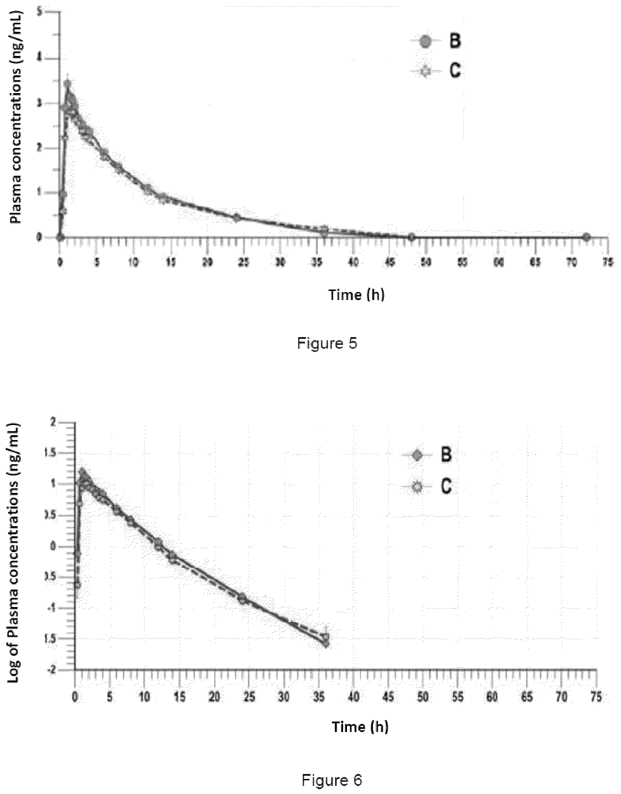

The average pharmacokinetic profile of standard error betamethasone on arithmetic and semi-log scale for comparative B vs C. Where B is: reference drug (betamethasone); and C is: test drug (desloratadine / betamethasone) are shown in Figures 5 and 6 below.

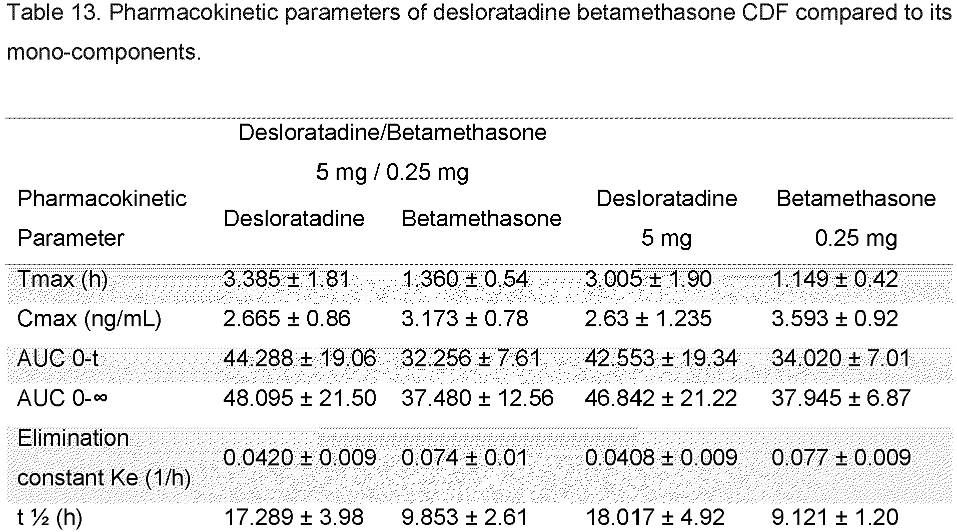

All the criteria used for non-pharmacokinetic interaction were met, since for each of the contrasts (C vs A and C vs B) where: A is Desloratadine monotherapy, reference drug, B is Betamethasone monotherapy, reference drug, C is Desloratadine / Betamethasone fixed dose combination, test drug, it has that in the variables Ln (C max), Ln (ABC ₀₋ₜ) and Ln (ABC ₀₋∞).
- The lower limit of the classical range (90% Cl) is greater than 80 and the upper limit of the classical range is less than 125%,
- The classical 95% confidence interval does not have values outside the 80-125% range.
- The p-values associated with the Schuirmann test for p 80 are less than .05 (p ≤ .005),
- The p-values associated with the Schuirmann test for p 80 are less than .05 (p ≤ .005),
- The Anderson Hauck test provides p-values much lower than .05, (p ≤ .01), so it is possible to reject the bioinequivalence hypothesis (a = .05).

In addition, we have that the powers were adequate, greater than .90 for the three variables under study, Ln (C max), Ln (AUC 0-t) and Ln (AUC 0 -∞), in both contrasts.

From the above, it is concluded that there is no pharmacokinetic interaction when Desloratadine is administered in combination with Betamethasone.

This study was conducted in accordance with the Official Mexican STANDARD NOM 177 SSA1 2013, Good Clinical Practices, the General Health Law in Research Matters.

### Advantages and applications of the invention

The present invention describes an optimized method that allows the combined manufacture of the drugs desloratadine and betamethasone in a single dose of 5.0/0.25mg respectively in one solid, stable, immediate-release dosage form. Thus, the present invention solves a set of important technological challenges due to physicochemical properties, for example; it has been achieved to stabilize desloratadine since it presents degradation under humid and temperature conditions. The absorption of light and moisture is moderated through the use of excipients that allow the protection of drugs without affecting their release and secondary materials that ensure their stability, ensuring the obtaining of a stable product that would otherwise be impossible to obtain or formulate.

The composition of the present invention is intended for the treatment and control of allergic and inflammatory processes in a subject selected from: severe symptoms of atopic dermatitis, angioedema, urticaria, seasonal and perennial allergic rhinitis, food and drug allergic reactions, allergic asthma, and allergic reactions secondary to insect bites.

### References

Back HM, Song B, Pradhan S, ChaeJW, Han N, Kang W, Chang MJ, Zheng J, Kwon KI, Karlsson MO, Yun HY. A mechanism-based pharmacokinetic model of fenofibrate for explaining increased. drug absorption after food consumption. BMC Pharmacology and Toxicology (2018) 19:4, pp. 1-10.

Chou YC, Wang YK, Charng MJ, Ueng YF. Determination of serum atorvastatin concentrations in lipid-controlling patients with and without myalgia syndrome. Journal of Food and Drug Analysis, Volume 21, Issue 2, June 2013, Pages 147-153.

Food and Drug Administration (FDA), Center for Drug Evaluation and Research (CDER). Clinical Drug Interaction Studies -Cytochrome P450 Enzyme- and Transporter-Mediated Drug Interactions Guidance for Industry. January 2020 Clinical Pharmacology.

Lins RL, Matthys KE, Verpooten GA, Peeters PC, Dratwa M, Stolear JC, Lameire NH. Pharmacokinetics of Atorvastatin and Its Metabolites After Single and Multiple Dosing in Hypercholesterolaemic Haemodialysis Patients. Nephrol Dial Transplant. 2003 May;18(5):967-76.

LIPITOR^{®} (atorvastatin calcium tablets), Product monograph 10 mg, 20 mg, 40 mg and 80 mg atorvastatin. Lipid metabolism regulator. Submission Control No: 237270. Pfizer Ireland Pharmaceuticals Upjohn Canada ULC, Licensee ^{©} Upjohn Canada ULC, 2020. Date of Revision: April 30, 2020; page 27.

Moffat AC, Osselton MD, Widdop B. Clarke's Analysis of Drugs and Poisons in pharmaceuticals, body fluids and postmortem material. Fourth edition, Pharmaceutical Press 2011: 42

Reddy P, Ellington D, Zhu Y, Zdrojewski I, Parent SJ, Harmatz JS, Derendorf H, Greenblatt DJ, Browne J. Serum concentrations and clinical effects of atorvastatin in patients taking grapefruit 25 juice daily. British Journal of Clinical Pharmacology © 2011, 72:3 / 434-441.

Wong B.A. Focus on Statin Research. New Biomedical Books, New York, 2006: 112-130.

Yamazaki M, Li B, Louie SW, Pudvah NT, Stocco R, Wong W, Abramovitz M, Demartis A, Laufer 30 R, Hochman JH, Prueksaritanont T, Lin JH. Effects of Fibrates on Human Organic Anion-Transporting Polypeptide 1B1-, Multidrug Resistance Protein 2- And P-glycoprotein-mediated Transport. Xenobiotics. 2005 Jul;35(7):737-53.

## Claims

1. A pharmaceutical composition **characterized by** comprising: (a) desloratadine, or an equivalent amount of a salt thereof, in a pharmaceutically acceptable amount of 5.00 mg and (b) betamethasone, or an equivalent amount of a salt thereof, in a pharmaceutically acceptable amount of 0.25 mg, and (c) a pharmaceutically acceptable amount of pharmaceutically acceptable excipients and/or pharmaceutical carriers; and wherein further said composition, stable, immediate release and in solid form.

2. As per claim 1, ideally, desloratadine is in its free form in the pharmaceutical composition.

3. As per claim 1, ideally, betamethasone is in its free form in the pharmaceutical composition.

4. According to claims 1 to 3, the pharmaceutical acceptable excipients and/or carriers of the pharmaceutical composition include diluents, disintegrants, adsorbents, surfactants, lubricants, and coatings.

5. According to claim 4, the diluents of the pharmaceutical composition are, preferably, PH102 microcrystalline cellulose in a pharmaceutically acceptable amount in the range of 20 to 90%; pregelatinized starch in a pharmaceutically acceptable amount in the range of 5.0 to 75% and anhydrous calcium dibasic phosphate in a pharmaceutically acceptable amount in the range of 10 to 30%.

6. According to claim 4, the disintegrant of the pharmaceutical composition, is, preferably, crospovidone in a pharmaceutically acceptable amount in the range of from 2.0 to 5.0%.

7. According to claim 4, the adsorbent of the pharmaceutical composition is, preferably, aluminum metasilicate and magnesium in a pharmaceutically acceptable amount in the range of from 0.5 to 90%.

8. According to claim 4, the surfactant of the pharmaceutical composition is, preferably, sodium lauryl sulphate in a pharmaceutically acceptable amount in the range from 0.5 to 2.5%.

9. The pharmaceutical composition, according to claim 4, contains Sodium stearyl fumarate as the lubricant in a pharmaceutically acceptable amount in the range from 0.5 to 2.0%.

10. The coating of pharmaceutical composition, according to claim 4, is a moisture barrier coating.

11. The moisture barrier coating of the pharmaceutical composition, according to claim 10 is, preferably, YS-1-7003 White opadry in a pharmaceutically acceptable amount of 3.85%.

12. The pharmaceutical composition, according to any of claims 1-11, is presented in a solid dosage form selected from the group consisting of a tablet, caplet, granules, lozenges, pills.

13. The pharmaceutical composition, according to claim 12, is preferably presented in its free tablet or coated pill form.

14. Use of the pharmaceutical composition of any of claims 1-13 for the manufacture of a drug product useful in treating allergic and inflammatory processes in a subject.

15. The use according to claim 14, wherein the allergic and inflammatory processes are selected from: severe symptoms of atopic dermatitis, angioedema, urticaria, seasonal and perennial allergic rhinitis, food and drug allergic reactions, allergic asthma, and allergic reactions secondary to insect bites.
